# EUROPÄISCHE PATENTANMELDUNG

(11) **EP 0 582 155 A1**
(43) Veröffentlichungstag der Anmeldung: **09.02.1994**
(21) Anmeldenummer: 93111855.8
(22) Anmeldetag: 23.07.1993
(51) Int. Cl.: A61M 25/00, A61M 25/10

(54) **Blasenkatheter**

(30) Priorität: 29.07.1992 DE 4225067
(71) Anmelder: Kraus, Kurt, D-24229 Strande (DE)
(72) Erfinder: Kraus, Kurt, D-24229 Strande (DE)
(74) Vertreter: Schwan, Gerhard, Dipl.-Ing.

(57) **Zusammenfassung**

Die vorliegende Erfindung betrifft einen Blasenkatheter mit einem fluidfüllbaren Ballon (11, 31) im Bereich des distalen Endes des Katheters (10) und einem den Katheter (10) ringförmig umgebenden Schwellkörper (12, 32), der proximal von dem Ballon angeordnet ist.

Es ist Aufgabe der Erfindung, diesen Katheter so weiterzubilden, daß eine hohe Dichtigkeit und ein sicherer Sitz des Katheters in der Harnröhre erreicht wird.

Diese Aufgabe wird dadurch gelöst, daß der Schwellkörper (12, 32) unmittelbar hinter dem Ballon (11, 31) angeordnet ist, daß der Ballon und der Schwellkörper an einen gemeinsamen Füllkanal (15) angeschlossen sind, und daß die Dehnungseigenschaften des Ballons und des Schwellkörpers derart aufeinander abgestimmt sind, daß sich beim Aufblasen zunächst der Ballon und erst danach der Schwellkörper ausdehnt.

## Beschreibung

Die vorliegende Erfindung betrifft einen Blasenkatheter gemäß dem Oberbegriff des Anspruchs 1.

Etwa 80 % der Erdbevölkerung, vornehmlich ältere männliche Personen, haben mit zunehmendem Alter Beschwerden an den Harnwegen, insbesondere der Prostata und an der Blase. Auch weibliche Personen erkranken im fortschreitenden Alter häufiger an der Blase und den Harnwegen, so daß im Einzelfall eine Operation indiziert ist, um die eingetretenen Schäden zu beheben. Für die Diagnostik des Einzelfalles wird vom Urologen in der Regel ein Endoskop in die Harnröhre eingeführt, um die Krankheit diagnostizieren zu können und entsprechend nachfolgende Behandlungs- oder auch Operationsmöglichkeiten festzulegen. Wenn ein Endoskop oder ähnliches Gerät, in der Regel mit Gleitmittel, in die Harnröhre eingeführt wird, wird diese geweitet und häufig auch beschädigt, was der Patient spätestens dann merkt, wenn er das Gefühl hat, Wasser lassen zu müssen. Durch den salzhaltigen Urin treten beim Durchfluß durch die Harnröhre starke Schmerzen brennender Art auf, die ein Zusammenkrampfen der Blase zur Folge haben. Dieser Harndrang ist nicht nur äußerst schmerzhaft, sondern verzögert auch den Heilungsprozeß nach einer Operation.

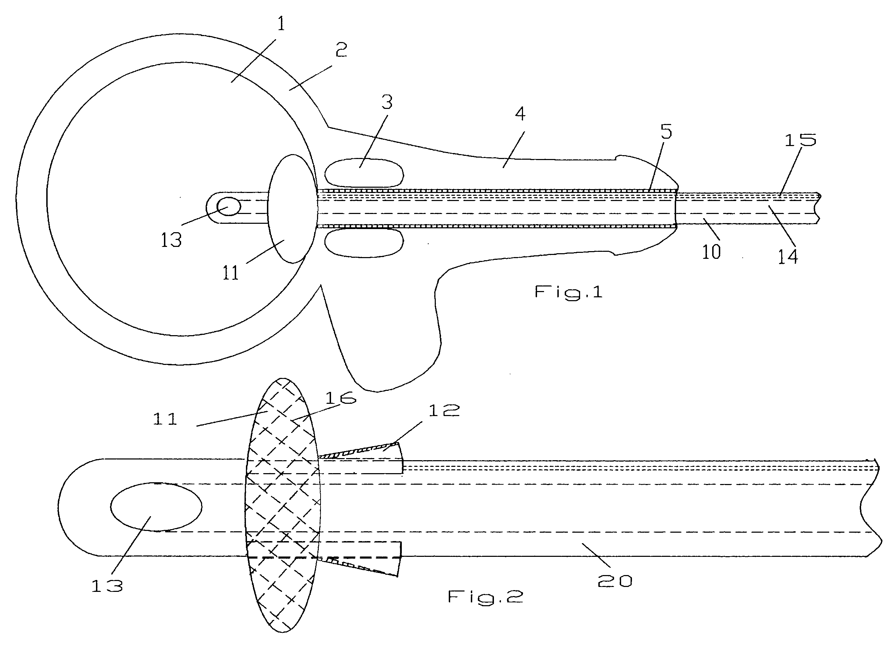

Fig. 1 zeigt schematisch einen Blasenkatheter 10, wie er derzeit üblichenweise verwendet wird. Ein solcher bekannter Katheter wird durch die Harnröhre 5 im Penis 4 derart an der Prostata 3 vorbei vorgeschoben, daß eine mit einem Katheterkanal 14 in Verbindung stehende Öffnung 13 am distalen Ende des Katheters10 im Innern der Blase 1 zu liegen kommt. Die Blasenwand ist mit 2 bezeichnet. Der Katheter 10 weist hinter der Öffnung 13 nahe seinem distalen Ende einen Ballon 11 auf. Der Ballon 11 kann über eine Versorgungsleitung 15, die sich parallel zu dem Kanal 14 in Längsrichtung durch den Katheter 10 hindurch erstreckt, wahlweise aufgeweitet oder in einen das Durchführen des Katheters durch die Harnröhre erlaubenden entleerten Zustand gebracht werden. Der in der Blase 1 aufgeweitete Ballon 11 soll den Katheter in einer Stellung halten, in welcher die Öffnung 13 innerhalb der Blase liegt; ein unbeabsichtigtes Herausrutschen des distalen Katheterendes aus der Blase 1 soll auf diese Weise verhindert werden. Eine gewisse Abdichtung der Blase 1 gegenüber der Harnröhre 5 bei eingeführtem Katheter soll, wenn überhaupt, durch eine den anatomischen Gegebenheiten der Harnröhre angepaßte Wahl des Durchmessers des Katheters erreicht werden. Dadurch läßt sich jedoch nicht verhindern, daß in einer für den Patienten oft äußerst schmerzhaften Weise Urin zwischen die Umfangswand des Katheters 10 und die Innenwand der Harnröhre 5 gelangt, wenn beispielsweise nach einer Operation Blutcoagel den Katheterausgang verstopft und der Urin zwischen Katheter und Harnröhre ins Freie drückt.

Ferner ist aus der DE 40 24 950 A1 ein Harnröhren-Verweilkatheter mit zwecks Inkontinenzsteuerung magnetisch betätigbarem Drainageventil bekannt. Dieser Katheter hat ähnlich wie der vorstehend an Hand der Fig. 1 erläuterte Katheter an seinem distalen Ende einen aufblasbaren Ballon, der sich gegen die Blasenwand abstützt. Weiterhin hat dieser Katheter eine aufblasbare Harnröhrenmanschette, die aus Silikon besteht und in einem vorgegebenen Abstand vom Blasenballon mit dem Umfang des Katheters verbunden ist. Ballon und Harnröhrenmanschette lassen sich über getrennte Kanäle aufblasen. Auch bei dieser Anordnung kann nicht verhindert werden, daß in der Blase befindliche Medien, insbesondere Urin, mindestens in dem Raum zwischen dem Blasenballon und der Harnröhrenmanschette mit der Harnröhrenwand in Kontakt kommen, was an diesen Stellen starke Schmerzen hervorrufen kann, wenn z.B. die Harnröhre bei häufigem Einführen von Kathetern entzündet oder beschädigt ist.

Es ist daher Aufgabe der Erfindung, einen Blasenkatheter der eingangs genannten Art zu schaffen, der eine weitgehend schmerzfreie Katheterisierung der Blase zuläßt.

Bei einem Blasenkatheter mit den Merkmalen des Oberbegriffs des Anspruchs 1 wird diese Aufgabe erfindungsgemäß dadurch gelöst.
daß der Schwellkörper unmittelbar hinter dem Ballon angeordnet ist,
daß der Ballon und der Schwellkörper an einen gemeinsamen Füllkanal angeschlossen sind, und
daß die Dehnungseigenschaften des Ballons und des Schwellkörpers derart aufeinander abgestimmt sind, daß sich beim Aufblasen zunächst der Ballon und erst danach der Schwellkörper ausdehnt.

Vorteilhafte Weiterbildungen der Erfindung sind Gegenstand der Unteransprüche.

Bei dem Blasenkatheter nach der Erfindung ist der Schwellkörper, z.B. eine Harnröhrenmanschette, unmittelbar hinter dem Ballon (an dessen proximaler Seite) angeordnet und zusammen mit diesem an einen gemeinsamen Füllkanal angeschlossen. Die Dehnungseigenschaften des Ballons und des Schwellkörpers sind dabei derart aufeinander abgestimmt, daß sich beim Aufblasen zunächst der Ballon und erst anschließend das Schwellsystem ausdehnt. Hierdurch wird eine hervorragende Absicherung der gesamten Harnröhre zur Blase hin gewährleistet. Gegen das Eindringen von Urin, Spülflüssigkeit oder dergleichen in den Raum zwischen Katheter und Harnröhre ist wirkungsvoll vorgesorgt. Dadurch, daß der Schwellkörper unmittelbar hinter dem Blasenballon angeordnet ist, wird auch ein Eindringen von Urin, Spülflüssigkeit oder dergleichen zwischen Ballon und Schwellkörper verhindert. Durch die gegenüber dem Blasenballon verzögerte Aufblähung des Schwellkörpers wird erreicht, daß zuerst der Katheter mit Bezug auf die Harnröhre durch den Blasenballon in korrekter räumlicher Lage fixiert wird und daß anschließend durch Aufweiten des Schwellkörpers eine effektive, die gesamte Harnröhre schützende Abdichtung erfolgt. Diese zeitliche Verzögerung zwischen dem Aufblasen von Ballon und Schwellkörper läßt sich z.B. durch unterschiedliche Materialstärken der Wände des Blasenballons und des Schwellkörpers erzielen.

Vorzugsweise ist der Schwellkörper in der Art einer zum proximalen Ende hin konisch aufgeweiteten Manschette ausgebildet, die mit ihren radial äußeren Enden dicht an der Harnröhre anliegt. Hierdurch wird wirksam verhindert, daß Flüssigkeit, die den Bereich zwischen Blase und Blasenballon passiert hat, in die Harnröhre gelangen kann.

Der Ballon ist in einer vorteilhaften Weiterbildung der Erfindung mit einer Gitterstruktur umgeben oder aus einem Material mit einer definierten maximalen Dehnung hergestellt, die den aufgeblasenen Zustand des Blasenballons eindeutig definiert. Hierdurch wird erreicht, daß zuerst der Blasenballon bis zu seiner vollen maximalen Größe aufgeblasen wird und erst anschließend das Aufblasen des Schwellkörpers erfolgt. Dies ist eben nur dann möglich, wenn der Druck für die maximale Ausdehnung des Blasenballons eindeutig definiert ist.

Der Ballon und der Schwellkörper können mit Gas oder einer Flüssigkeit aufgeblasen werden. Durch den großflächigen Schwellkörper findet keine Punktbelastung des Gewebes im Bereich der Harnröhre statt, und ein Geweberückgang im Zeitpunkt eines normalen Operationsvorganges ist somit nicht zu erwarten. Weiterhin ist durch die Anordnung des Schwellkörpers, der eine hervorragende Dichtung zur Harnröhre darstellt, gewährleistet, daß der Katheter in seiner Position so lange beharrt und dicht ist, bis das Fluid, z.B. eine Kochsalzlösung, aus dem Ballon und dem Schwellkörper entfernt ist. Durch die gekoppelte Füllung und Entleerung des Ballons und des Schwellkörpers ist der Blasenkatheter gemäß der vorliegenden Erfindung einfach handhabbar.

Vorzugsweise weist der Schwellkörper unterschiedliche Materialstärken in unterschiedlichen radialen Abständen vom Katheter auf, so daß sowohl die verstärkte Ausführung im katheternahen Bereich eine feste Abdichtung als auch durch die schwächere Ausbildung im harnröhrennahen bzw. an der Harnröhre anliegenden Bereich eine sehr schonende Abdichtung gewährleistet wird.

Die Erfindung wird nachfolgend beispielsweise anhand der schematischen Zeichnungen beschrieben. In dieser zeigen:
- Fig. 1: einen Längsschnitt eines durch die Harnröhre eingeführten Blasenkatheters bekannter Art mit einem aufblasbaren Ballon, ohne Dichtfunktion,
- Fig. 2: in größerem Maßstab das distale Ende eines erfindungsgemäßen Blasenkatheters mit einem Schwellkörper,
- Fign. 3, 4 und 5: die Abfolge des Aufblasvorgangs des Ballons und Schwellkörpers beim Blasenkatheter der Fig. 2, und
- Fign. 6, 7 und 8: den Aufblasvorgang bei einer anderen erfindungsgemäßen Ausführungsform eines Blasenkatheters mit Ballon und Schwellkörper.

Der in den Fign. 2 bis 5 veranschaulichte Blasenkathether 10 weist ähnlich wie der Katheter gemäß Fig. 1 an seinem distalen Ende eine Öffnung 13 des durch den Katheter in Längsrichtung hindurchführenden Kanals 14 auf. Hinter der Öffnung 13 (d.h. in proximaler Richtung mit Bezug zur Öffnung 13) sitzt ein Ballon 11. Unmittelbar hinter dem Ballon 11 ist ein Schwellkörper 12 angeordnet, der in Fig. 2 ebenso wie der Ballon 11 im aufgeblasenen Zustand dargestellt ist.

Der Schwellkörper 12 hat die Form einer Manschette, die sich beim Aufblasen an ihrem proximalen Ende radial von der Wandung 20 des Katheters 10 wegspreizt (Fig. 2). Dieser in Form einer Harnröhrenmanschette ausgebildete Schwellkörper 12 dient der sicheren Abdichtung der Harnröhre 5 gegen das Eindringen von Urin, salzhaltiger Spülflüssigkeit oder dergleichen. In dem Katheter 10 verläuft ferner die Versorgungsleitung 15, durch die der vorzugsweise aus Gummi bestehende Ballon 11 und der Schwellkörper 12 entweder mit einer Kochsalzlösung oder mit einem Gas bzw. anderen Flüssigkeiten gefüllt werden können.

Wie in Fig. 2 zu sehen ist, ist der Ballon 11 von einer Netzstruktur 16 umgeben, die die maximale Größe des Ballons beim Aufblasvorgang eindeutig festlegt. Der Ballon 11 und der Schwellkörper 12 sind über einen gemeinsamen Fluidkanal miteinander verbunden.

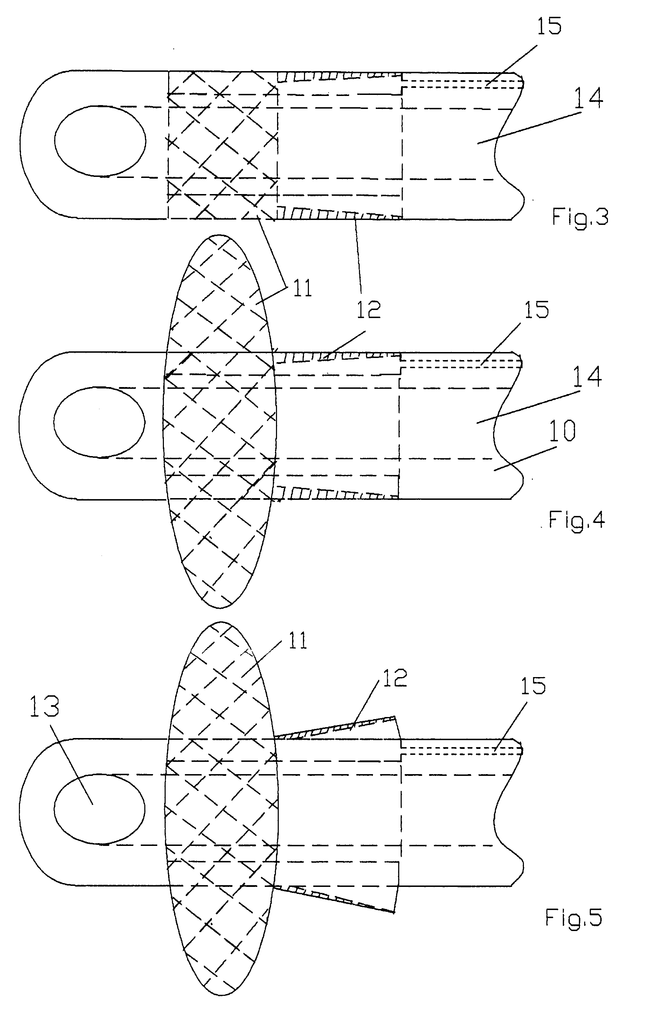

Die Vorgänge beim Aufblasen von Ballon 11 und Schwellkörper 12 sind in den Figuren 3 bis 5 dargestellt. Fig. 3 zeigt das distale Ende des Blasenkatheters 10 aus Fig. 2 beim Einführen in die Blase. Es ist zu erkennen, daß der Außendurchmesser des Katheters 10 sowohl im Bereich des Ballons 11 als auch im Bereich des Schwellkörpers 12 gegenüber dem Katheter-Außendurchmesser im übrigen Teil der Längsabmessung des Katheters derart reduziert ist, daß der Außenumfang des leeren Ballons und des leeren Schwellkörpers mindestens näherungsweise bündig mit dem in Axialrichtung daran anschließenden Teil des Außenumfangs des Katheters ist.

Wenn der Blasenkatheter 10 genug eingeschoben ist, was dem Arzt z. B. durch Abtropfen von Blasenurin angezeigt wird, wird der intravesikal liegende Gummiballon 11 über die Leitung 15 mit beispielsweise 5 bis 10 cm³ Kochsalzlösung aufgeblockt, und der Katheter 10 wird durch einen leichten Zug an den Blasenboden 2 plaziert. Die Größe des Katheters, dessen Umfang in Charrier (1 Charr. = 1/3 mm) angegeben wird, richtet sich nach den anatomischen Verhältnissen der Harnröhre sowie dem Grund zum Katheterismus. Es werden Katheter in den Größen von ca. Charr. 12 bis 27 und größer eingesetzt. Die Katheter selbst können hart oder weich sein und aus Kautschuk, elastischem Silikon als auch aus Edelstahl bestehen, was je nach Funktion und Einsatz festgelegt wird. Der in den Figuren 2 bis 5 dargestellte Katheter ist ein Dauerkatheter. Der Katheter ist im aufgeblockten Zustand des Ballons 11 in Fig. 4 dargestellt.

Nach richtiger Plazierung des Katheters wird über die Versorgungsleitung 15 weiter Kochsalzlösung zugeführt. Die maximale Größe des Ballons 11 ist durch die Gitterstruktur 16 festgelegt. Nach Erreichen dieser Größe wird nun der Schwellkörper 12 aufgeblockt, der sich in Art einer konisch abgespreizten Manschette an die Harnröhre anlegt. Das Dehnungsverhalten ist hierbei so gewählt, daß der Schwellkörper 12 erst aufgeblasen bzw. aufgeblockt wird, wenn der Ballon 11 seine maximale Größe erreicht hat. Dies kann sowohl durch entsprechend gewählte Materialstärken als auch durch Vorrichtungen wie z.B. die Gitterstruktur 16 erreicht werden. Weiterhin kann der Durchmesser der Fluidverbindungen zwischen Ballon und Schwellkörper zur Steuerung des Dehnungsverhaltens von Ballon 11 und Schwellkörper 12 verwendet werden.

Die Materialstärke des Schwellkörpers 12, der in Art einer Manschette ausgebildet ist, nimmt von seinem an dem Ballon 11 anliegenden distalen Ende zu seinem proximalen, radial von dem Katheter abstehenden und an der Harnröhre anliegenden Ende hin ab. Hierdurch wird erreicht, daß zwar der Schwellkörper 12 kräftig von dem Katheter 10 in Richtung auf die Harnröhre vorgespreizt wird, wegen der geringen Materialstärke in dem an der Harnröhre anliegenden Bereich jedoch sanft an der Harnröhre anliegt, wodurch einer Beschädigung bzw. Entzündung der Harnröhre im Anlagebereich des Schwellkörpers 12 vorgebeugt wird. Durch den großflächigen Schwellkörper 12 wird das Gewebe an der Harnröhre nicht punktartig belastet, und ein Geweberückgang im Zeitpunkt eines operativen Eingriffs ist nicht zu erwarten. Durch die Anordnung und richtige Auslegung des Schwellkörpers 12, der zugleich eine hervorragende Dichtung zur Harnröhre darstellt, ist weiterhin gewährleistet, daß der Katheter in seiner Position so lange beharrt und dicht ist, bis die Kochsalzlösung über die Versorgungsleitung 15 aus Ballon 11 und Schwellsystem 12 entfernt ist.

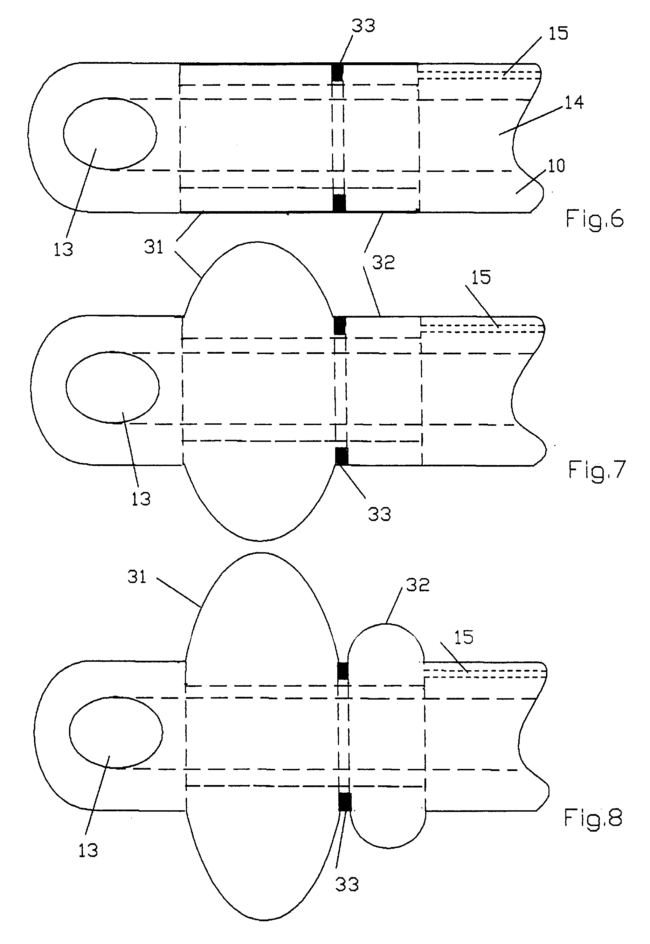

Eine weitere Ausführungsform der Erfindung ist in den Fign. 6 bis 8 veranschaulicht. Der Katheter selbst entspricht dabei dem in den Figuren 2 bis 5 dargestellten Katheter, wobei identische Teile mit identischen Bezugszeichen versehen sind.

Der Ballon 31 und der Schwellkörper 32 sind als ein durch einen Wulst 33 abgetrennter Ballon ausgebildet, wobei der manschettenartige Schwellkörper am Ende des distalen Drittels des Gesamtballons angeordnet ist, so daß Ballon 31 und Schwellkörper 32 ein Längenverhältnis von etwa 2 zu 1 aufweisen. Der nach innen liegende Wulst 33 kann aus dem gleichen Manschettenmaterial wie Ballon 31 und Schwellkörper 32 hergestellt sein. Beim Aufblasen (Fign. 7 und 8) dehnt sich zuerst wie bei einem Luftballon derjenige Teil aus, der die größte zusammenhängende Fläche aufweist. Dies ist der Ballon 31. Erst wenn der über die Versorgungsleitung 15 zugeführte Druck zum Aufblocken von Ballon und Schwellkörper durch die Dehnungsgrenze des Ballonmaterials stark ansteigt, wird auch der Schwellkörper 32 mit der kleineren Fläche aufgeblasen. Dieser letzte Vorgang erfolgt wie bereits anhand der Fign. 2 bis 5 beschrieben nach richtiger Positionierung des Katheters durch Zug am Katheter 10 bei aufgeblasenem Ballon 31 (Fig. 7). Beim Aufblasen des Schwellkörpers 32 werden wiederum eine sehr gute Abdichtung der Harnröhre und eine gute Positionierung des Katheters bewirkt.

Bei aufgeblasenem Ballon und Schwellkörper kann bei keiner der vorstehend dargestellten Ausführungsformen Salzlösung oder Urin in die Harnröhre eintreten. Der Heilungsprozeß wird daher verbessert, und das Auftreten von Blasenkrämpfen aufgrund von Schmerzen wird weitgehend ausgeschaltet.

## Patentansprüche

1. Blasenkatheter mit einem fluidfüllbaren Ballon (11, 31) im Bereich des distalen Endes des Katheters (10) und mit einem den Katheter (10) ringförmig umgebenden Schwellkörper, der proximal mit Bezug auf den Ballon (11) angeordnet ist, **dadurch gekennzeichnet,**
daß der Schwellkörper (12, 32) unmittelbar hinter dem Ballon (11, 31) angeordnet ist,
daß der Ballon (11,31) und der Schwellkörper (12, 32) an einen gemeinsamen Füllkanal (15) angeschlossen sind, und
daß die Dehnungseigenschaften des Ballons (11, 31) und des Schwellkörpers (12, 32) derart aufeinander abgestimmt sind, daß sich beim Aufblasen zunächst der Ballon (11, 31) und erst danach der Schwellkörper (12, 32) ausdehnt.

2. Blasenkatheter nach Anspruch 1, dadurch gekennzeichnet, daß der Schwellkörper (12) in der Art einer sich konusförmig aufweitenden Manschette ausgebildet ist, die zu ihrem proximalen Ende hin von dem Katheter (10) radial nach außen absteht.

3. Blasenkatheter nach Anspruch 2, dadurch gekennzeichnet, daß die Materialstärke des Schwellkörpers (12) zu dessen proximalem Ende hin abnimmt.

4. Blasenkatheter nach Anspruch 2 oder 3, dadurch gekennzeichnet, daß der Schwellkörper (12) zu seinem proximalen Ende hin zugespitzt ist.

5. Blasenkatheter nach einem der vorhergehenden Ansprüche, dadurch gekennzeichnet, daß der Ballon (11) von einer Netzstruktur (16) umgeben ist, die die maximale Größe des Ballons (11) festlegt.

6. Blasenkatheter nach einem der vorhergehenden Ansprüche, dadurch gekennzeichnet, daß die Materialstärke des Schwellkörpers (12) größer als die Materialstärke des Ballons (11) ist.

7. Blasenkatheter nach einem der vorhergehenden Ansprüche, dadurch gekennzeichnet, daß der Außendurchmesser des Katheters (10) im Bereich des Ballons (11, 31) und/oder des Schwellkörpers (12, 32) derart reduziert ist, daß der Außenumfang des Ballons und/oder des Schwellkörpers im nicht aufgeblasenen bzw. geblockten Zustand mindestens näherungsweise bündig mit dem Außenumfang des Katheters (10) ist.

8. Blasenkatheter nach einem der vorhergehenden Ansprüche, dadurch gekennzeichnet, daß der Ballon (31) und der Schwellkörper (32) durch einen mittels eines Wulstes (33) unterteilten gemeinsamen Ballon gebildet sind.
